Europäisches Patentamt

European Patent Office

Office européen des brevets

(19)

(11) Publication number: **0 225 759 B1**

# (12) EUROPEAN PATENT SPECIFICATION

(45) Date of publication of patent specification: **27.05.92**   (51) Int. Cl.⁵: **A61K  37/66**

(21) Application number: **86309134.4**

(22) Date of filing: **21.11.86**

(54) **Interferon compositions.**

(30) Priority: **11.12.85 US 807887**

(43) Date of publication of application:
**16.06.87 Bulletin  87/25**

(45) Publication of the grant of the patent:
**27.05.92 Bulletin  92/22**

(84) Designated Contracting States:
**AT BE CH DE ES FR GB GR IT LI LU NL SE**

(56) References cited:
**EP-A- 0 051 873**
**EP-A- 0 107 498**
**EP-A- 0 146 903**

**Nucleid Acids research 1981, 9/22). 6153**

(73) Proprietor: **SCHERING CORPORATION**
**2000 Galloping Hill Road**
**Kenilworth New Jersey 07033(US)**

(72) Inventor: **Leibowitz, Paul Jason**
**185 Prospect Avenue Apt 2N**
**Hackensack New Jersey 07601(US)**

(74) Representative: **Ritter, Stephen David et al**
**Mathys & Squire 10 Fleet Street**
**London EC4Y 1AY(GB)**

**Description**

This invention relates to interferon compositions and in particular to combinations of (a) human gamma interferon (IFN-γ), sometimes referred to as human immune interferon, and (b) synthetic abbreviated leukocyte interferon species (IFN-α), which species features a "sub-segment" defined as delta-4-alpha-2 (Bgl II-I) derived from an alpha-2 sequence that is joined to a segment defined as (Bgl II) alpha-I derived from an alpha-I sequence. These combinations display greater antiproliferative effects against human tumour cells and greater antiviral activity than could be expected from their individual activities.

There are a number of interferon species made by recombinant techniques wherein two or more leukocyte interferon segments are combined by use of now-standard recombinant DNA methods. Such species are disclosed in U.S. patents 4,456,748 and 4,4I4,I50 as well as in EP-A-0 051 873 and Weck et al., Nucleic Acids Research 9, 6I53 (I98I). However, there is no disclosure of a delta-4 α-interferon species, i.e. one lacking the first four amino acids.

EP-A-0 107 498 discloses that combinations of gamma interferon and, inter alia, "hybrid interferons" such as those disclosed by Weck et al and in EP-A-0 051 873 display "synergistic" biological activity as evidenced by antiproliferative effects against the human melanoma cell line Hs294T. However, there is no disclosure of a delta-4 species.

EP-A-0 146 903 discloses a synthetic abbreviated interferon which exemplifies the interferon species suitable for use in this invention, i.e. delta-4 alpha-2 (Bgl II-I) derived from an alpha-2 sequence that is joined to a segment defined as (Bgl II) alpha-I derived from an alpha-I sequence.

This invention is directed to novel synergistic pharmaceutical compositions comprising human gamma interferon (IFN-γ) and the synthetic abbreviated interferon species disclosed in EP-A-0 146 903, in a pharmaceutically pure admixture. The synthetic abbreviated interferon species used in this invention is identified in said EP-A-0 146 903 as delta-4 alpha-2 (Bgl II-I) derived from an alpha-2 sequence that is joined to a segment defined as (Bgl II) alpha-I derived from an alpha-I sequence. For convenience it will be referred to herein simply as delta-4 interferon.

Gamma interferon, both naturally occurring and recombinant, and the delta-4 interferon, a synthetic abbreviated interferon species, which is made as disclosed in said EP-A-0 146 903, are both known to have anti-tumour activity, i.e. they manifest antiproliferative effects when used to treat tumour cells and they also are known to have antiviral effects. However, their individual effects are such that effective use requires doses so large that toxic side-effects occur. There is therefore a need for a means to achieve the same or better antiproliferative effects against tumours or the same or better antiviral effects with lower doses of the interferons used so that the toxic side effects are either eliminated or reduced to a tolerable level.

This invention provides compositions for treating susceptible tumours and susceptible viruses utilizing lower doses of gamma interferon and delta-4 interferon in combination than would be required if each were used alone.

More particularly, this invention is based on the discovery that a pharmaceutical composition containing an anti-neoplastically effective amount or an antivirally effective amount of a combination of gamma interferon and delta-4 interferon is more effective in treating susceptible tumours or susceptible viruses than would be expected from the potencies of the individual interferons.

Administration of the compositions or combinations of this invention can be by any of the accepted modes of administration for antineoplastic or antiviral interferons. These methods include oral, parenteral, topical, depot and transdermal. Subcutaneous or intravenous injection is preferred.

Depending on the intended mode of administration, the compositions containing the gamma and/or delta-4 interferons may be in the form of solid, semi-solid or liquid dosage forms, such as, for example, tablets, pills, capsules, powders for reconstitution, liquids, suspensions, or the like, preferably in unit dosage forms suitable for single administration of precise dosages.

The pharmaceutically acceptable carriers and excipients useful in this invention are conventional. There may be included other medicinal and pharmaceutical agents. Excipients which may be used include but are not limited to other proteins, such as human serum albumin or plasma preparations.

The amount of active compound administered will, of course, be dependent on the patient being treated, the severity of the affliction and the manner of administration in the judgment of the prescribing physician.

For solid compositions, conventional non-toxic solid carriers include, for example, pharmaceutical grades of mannitol, lactose, starch, or magnesium stearate. Liquid injectable pharmaceutically administrable compositions can, for example, be prepared by dissolution or dispersion in a carrier, for example water, saline, aqueous dextrose, glycerol, and ethanol, to thereby form a solution or suspension for injection. If desired, the pharmaceutical composition to be administered may also contain minor amounts of non-toxic auxiliary substances such as wetting or emulsifying agents, preservatives and pH buffering agents, for example sodium acetate or sorbitan monolaurate. Actual methods of preparing such dosage forms are known or will be apparent to those skilled in this art; for example, see Remingtons's Pharmaceutical Sciences, Mack Publishing Company, Easton, Pennsylvania, l5th Edition, l975. The composition or formulation to be administered will, in any event, contain a quantity of the active components in an amount effective to achieve the desired effect in the patient being treated.

The combination of gamma interferon and delta-4 interferon can contain a wide range of international reference units per ml (IRU/ml) of each interferon and still display a greater-than-additive effect against tumors or viruses; for example, the amount of gamma interferon administered can be from $0.02 \times 10^6$ IRU/m$^2$ to $2 \times 10^6$ IRU/m$^2$ and the amount of delta-4 interferon administered can be from $0.02 \times 10^6$ IRU/m$^2$ to $10 \times 10^6$ IRU/m$^2$.

On the basis of the tests reported herein one would also expect that the combination of interferons according to this invention will exhibit greater-than-additive effects against viruses such as the polio-like virus EMC and the rabies-like virus VSV.

The following illustrates the antiproliferative effects of combining gamma interferon with an abbreviated interferon species containing a delta-4 component.

MATERIALS AND METHODS

Cell Lines.

The human bladder carcinoma cell line RT4, Rigby et al., British J. Cancer 24, 746 (l970), and the human lung carcinoma cell line A2l82 (S.A. Aaronson, National Cancer Institute) were maintained in RPMI l640 medium supplemented with l0% heat-inactivated foetal calf serum, 2mM glutamine, penicillin and streptomycin (complete medium) at 37°C in a humidified $CO_2$ atmosphere. The cells were subcultured with a 0.25% trypsin (Worthington) - EDTA solution when the cells were approximately 80% confluent.

Interferons.

A delta-4 interferon which contains amino acids 5-62 of alpha-2 IFN and 64-l66 of alpha-l IFN (specific activity $1.0 \times 10^8$ IRU/mg) was diluted with phosphate buffered saline, pH 7.4, containing 3.5 mg/ml human serum albumin, aliquoted and stored at -70°C until used. Its specific activity was calculated using NIH natural $\alpha$-IFN WHO/NIH 69/l9 (specific activity $3 \times 10^6$ IRU/mg) as a standard. Recombinant gamma interferon (specific activity $4.0 \times 10^6$ IRU/mg) was stored undiluted at 4°C until used. The specific activity of the recombinant gamma IFN was calculated using NIH natural $\gamma$-IFN Gg23-90l-530 (specific activity $7 \times 10^5$ IRU/mg) as a standard.

Antiproliferative Assays.

Assays of cell growth inhibition were performed as previously described by Hubbell et al., Cancer Res. 44, 3252 (l984). Cells in log phase growth were used for the assay. On day -l, $1 \times 10^5$ cells were plated on 35mm Petri dishes and allowed to attach overnight. At time 0, the medium was aspirated, the plates washed once with Hanks' balanced salt solution (HBSS) and then re-fed with fresh medium either alone or containing the appropriate concentration of interferon (IFN). For experiments with the combination of this invention the cells were fed with fresh medium containing a mixture of the IFNs at the appropriate concentrations. After a 72 hr incubation period, the cells were counted in a Coulter counter, model $Z_{BI}$, as follows. The cells were washed once with HBSS, removed from the plates with trypsin and added to an equal volume of complete medium. The plates were rinsed once with complete medium which was combined with the cell suspension. The cells were diluted l0-fold with TBS (20 mM Tris, pH 7.5, l50 mM NaCl) for counting. The % control growth was calculated from the formula:

$$\frac{\text{Treated cells day 3 - Control cells day 0}}{\text{Control cells day 3 - Control cells day 0}} \times 100$$

Statistical Analysis.

3

Results were analyzed by linear regression analysis using the logarithm of the IFN concentration vs. the calculated % control growth. The statistical validity of each experiment was verified by the correlation coefficient (r) for each regression line. All r values were greater than 0.78. The amount of IFN needed to inhibit growth by 50% ($GI_{50}$) was derived from the regression analysis and used as a point of comparison (Hubbell et al., vide supra). Significance (p) values were calculated for the comparison of effectiveness of the INFs used individually by three-way analysis of variance and Scheffe's multiple contrast method; see Zar, Biostatistical Analysis, Prentice Hall Inc. l05-l07, l59-l6l, l90-l93 (l974).

The extent of the effect of the combined IFN treatments was calculated by the isobole method for a combination for drugs A and B (a standard method reported by Berenbaum, Advances in Cancer Research, 35 269 (l98l)) from the equation

$$\frac{A_c}{A_e} + \frac{B_c}{B_e} = D$$

where $A_c$ and $B_c$ are the doses of the drugs used in the combination treatment and $A_c$ and $B_e$ are the doses of the drugs used individually to give the same magnitude of effect as in the combination. If D<l the IFN combination gives more than an additive antiproliferative effect. If D = l the effect is merely additive, and if D>l the effect is antagonistic. The values for $A_c$ and $B_e$ were derived from a line generated by the dose-response curves obtained for each IFN individually on each cell line by using the average slope of multiple experiments. The Y-intercept was determined by using the value obtained in the experiments for each IFN alone. Interferon potentiation was calculated as the ratio $A_c/A_c$ where $A_c$ is the amount of the delta-4 interferon required to give the same effect as the combination containing also gamma interferon, and $A_c$ is the amount of the delta-4 IFN used in the combination. Duplicate or triplicate experiments allowed a calculation (by the one-sided Student's t test, Zar, vide supra) of the statistical significance values (p) of the combination indices (D) compared to the additive combination index of D = l.

RESULTS

The results are shown in the Tables which follow.

Antiproliferative Effect of IFNs.

RT4 and A2l82 cells were treated in a dose-response manner, range l00-5000 IRU/ml for delta-4 IFN, l0-500 IRU/ml for gamma IFN. RT4 cells were overall more sensitive to the antiproliferative effects of the IFNs than A2l82 cells.

## Table 1

### Relative Effectiveness of Delta-4 and Gamma Interferons as Antiproliferative Agents

| Cell Line | Interferon Species | $GI_{50}$[a] IRU/ml[b] |
|---|---|---|
| RT4 | Delta-4 | 1050 |
|  | Gamma | 14[c] |
| A2182 | Delta-4 | 4700 |
|  | Gamma | 390[c] |

a All values average of two experiments, unless otherwise noted.

b International reference units per milliliter.

c Average of three experiments.

Table I summarizes the dose-response results by comparing the $GI_{50}$ values for gamma and delta-4 subsegment IFNs. On an IRU/ml basis, the gamma IFN is significantly more effective than the delta-4 IFN in both RT4 and A2I82 cells.

Antiproliferative Effect of IFN Combinations.

All cell lines were treated with combinations of the gamma and the delta-4 IFN preparations to assess the potential for potentiated antiproliferative effects. In these experiments, doses of the IFNs were used which alone caused only limited inhibition of tumor cell growth. The potentiation in each experiment was calculated using a line generated from the individual treatments of the experiments and the average slope from independently generated dose-response curves.

## Table 2

Potentiated Antiproliferative Combinations of Delta-4 Interferon and Gamma Interferon in the Bladder Carcinoma cell Line RT 4

| IFN($A_c$) IRU/ml | Gamma IFN($B_c$) IRU/ml | %Control Growth | IFN($A_e$) IRU/ml | Gamma IFN($B_e$) IRU/ml | Combination Index(D) |
|---|---|---|---|---|---|
| **Experiment I** | | | | | |
| Delta-4 100 | 5.0 | 13.6 | >5,000 | 50 | <0.120 |
| **Experiment II** | | | | | |
| Delta-4 100 | 5.0 | −34.1 | >5,000 | >500 | <0.030 |
| **Experiment III** | | | | | |
| Delta-4 100 | 5.0 | −12.0 | >5,000 | >500 | <0.030 |

Table 2 summarizes the results of three experiments on RT4 cells using gamma IFN in combination with each of the delta-4 IFN preparations. Statistical analysis of the experimental combination index (D) values compared to D = I (additive effect) indicates that gamma IFN in combination with the delta-4 IFN studies inhibited tumor cell growth in a more-than-additive manner (p<0.05). For each combination, l00 IRU/ml of the delta-4 IFN was used; however, an amount of more than 5000 IRU/ml was needed when the delta-4 IFN was used alone, in order for it to be as effective as the combination. Thus, in all cases, the gamma IFN potentiated the delta-4 effect more than 50-fold.

## Table 3

Potentiated Antiproliferative Combinations of Delta-4 Interferon and Gamma Interferon in the Lung Carcinoma Cell Line A2182

| IFN(A) IRU/ml | Gamma IFN($B_c$) IRU/ml | %Control Growth | IFN($A_e$) IRU/ml | Gamma IFN($B_e$) IRU/ml | Combination Index (D) |
|---|---|---|---|---|---|
| **Experiment I** | | | | | |
| Delta-4 <br> 100 | 20 | 13.1 | >5,000 | 178 | <0.132 |
| **Experiment II** | | | | | |
| Delta-4 <br> 100 | 20 | 21.6 | >5,000 | >500 | <0.060 |
| **Experiment III** | | | | | |
| Delta-4 <br> 100 | 20 | 22.2 | >5,000 | 302 | <0.086 |

The data in Table 3 show that gamma interferon in combination with delta-4 interferon exhibits greater-than-additive effects in A2l82 cells. Statistical analysis indicates that all gamma interferon/delta-4 interferon combinations inhibit A2l82 cell growth in a more-than-additive manner (p<0.05). The gamma interferon potentiates the delta-4 interferon more than 50-fold.

Gamma Interferon Concentration and Magnitude of Potentiations.

In order to determine the contribution of gamma interferon to the potentiated antiproliferative effects seen in the delta-4 IFN/gamma IFN combinations, the amount of gamma IFN was varied over a 25-fold range in RT4 cells.

## Table 4

## Variation in Potentiated Antiproliferative Effect with Different Gamma Interferon concentrations in RT4 Cells

| IFN($A_c$) IRU/ml | Gamma IFN($B_c$) IRU/ml | %Control Growth | IFN($A_e$) IRU/ml | Gamma IFN($B_e$) IRU/ml | Combination Index(D) |
|---|---|---|---|---|---|
| Delta-4 | | | | | |
| 100 | 0.2 | 124.4 | 162 | 0.03 | 8.61 |
| 100 | 0.5 | 113.0 | 417 | 0.22 | 2.51 |
| 100 | 2.0 | 56.1 | >5,000 | 7.59 | <0.28 |
| 100 | 5.0 | 17.0 | >5,000 | 79.00 | <0.08 |

As seen in Table 4, the concentration of gamma IFN plays a significant role in the magnitude of potentiation obtained. Gamma IFN at 0.2 and 0.5 IRU/ml, in combination with the delta-4 IFN, shows a slight stimulation of cell growth, and a clear-cut antagonistic effect is thus seen at the lower gamma IFN concentrations. Potentiation is seen at 2.0 and 5.0 IRU/ml gamma IFN with the delta-4 IFN.

### DISCUSSION

The two cell lines studies here were inhibited in a dose-dependent manner by the various IFN preparations. In general, RT4 cells were more sensitive to delta-4 IFN than were A2l82 cells. When calculated on an IRU/ml basis, gamma IFN was more effective than the delta-4 IFN in both cell lines.

As the data in the Tables indicate, combinations of gamma IFN with the delta-4 IFN yielded more-than-additive antitumour effects in both cell lines. The antigrowth effects of the delta-4 IFN were potentiated more than 50-fold by the gamma IFN. Significantly more-than-additive antitumour effects were seen in the A2l82 cells, even though these cells were relatively resistant to delta-4 IFN alone. Use of various concentrations of the gamma IFN in RT4 cells demonstrated that potentiation occurred especially within a particular range of IFN concentrations. The potency of the delta-4 IFN also appeared to determine at what concentrations gamma IFN would potentiate. A relatively low concentration was used for all delta-4 IFN preparations (l00 IRU/ml) and potentiation was seen at a lower concentration of gamma IFN (2.0 IRU/ml) using the most potent delta-4 IFN preparation. In the experiments used in this application highly purified recombinant IFN preparations were used.

The results indicate that the clinical use of delta-4/gamma IFN combinations may be more successful than present-day single-IFN therapies. Significant decrease in the required dose of the IFNs has the advantage of minimizing toxic side effects during treatment. In addition, the range of patients responding to therapy should be significantly increased. These results indicate that combination therapy, in general, should greatly increase the range of sensitive tumors and, specifically, that delta-4/gamma IFN therapy may be effective in tumours presently resistant to these interferons and display antiproliferative effects where, previously, either delta-4 or gamma interferons were separately ineffective.

### Claims

1. A synergistic pharmaceutical composition comprising a combination of gamma interferon in a pharmaceutically pure admixture with a second interferon which is a delta-4 interferon.

2. A composition as claimed in claim 1 which is an injectible composition.

**3.** A composition as claimed in claim 1 or claim 2 comprising a pharmaceutically acceptable carrier.

**4.** A pharmaceutical composition as claimed in any of claims 1 to 3 comprising an antineoplastically effective amount of said combination.

**5.** A pharmaceutical composition as claimed in any of claims 1 to 3 comprising an antivirally effective amount of said combination.

**Revendications**

**1.** Composition pharmaceutique synergique comprenant une combinaison d'interféron gamma dans un mélange pharmaceutiquement pur avec un second interféron qui est l'interféron delta-4.

**2.** Composition selon la revendication 1, qui est une composition injectable.

**3.** Composition selon l'une quelconque des revendications 1 ou 2, comprenant un support pharmaceutiquement acceptable.

**4.** Composition pharmaceutique selon l'une quelconque des revendications 1 à 3, comprenant une quantité de ladite composition efficace comme agent anti-cancéreux.

**5.** Composition pharmaceutique selon l'une quelconque des revendications 1 à 3 comprenant une quantité de ladite composition efficace comme agent anti-viral.

**Patentansprüche**

**1.** Synergistische pharmazeutische Zusammensetzung, umfassend eine Kombination von gamma-Interferon in einer pharmazeutisch reinen Abmischung mit einem zweiten Interferon, das delta-4-Interferon ist.

**2.** Zusammensetzung nach Anspruch 1, die eine injizierbare Zusammensetzung ist.

**3.** Zusammensetzung nach Anspruch 1 oder Anspruch 2, umfassend einen pharmazeutisch annehmbaren Träger.

**4.** Zusammensetzung nach irgendeinem der Ansprüche 1 bis 3, umfassend eine antineoplastisch wirksame Menge der genannten Kombination.

**5.** Zusammensetzung nach irgendeinem der Ansprüche 1 bis 3, umfassend eine antiviral wirksame Menge der genannten Kombination.